# EUROPÄISCHE PATENTSCHRIFT

(11) **EP 2 802 301 B1**
(45) Veröffentlichungstag und Bekanntmachung des Hinweises auf die Patenterteilung: **03.08.2016**
(21) Anmeldenummer: 13706159.4
(22) Anmeldetag: 09.01.2013
(51) Int. Cl.: A61F 9/007

(54) **VORRICHTUNG ZUR VERWENDUNG IN DER GLAUKOMCHIRURGIE**
DEVICE FOR USE IN GLAUCOMA SURGERY
DISPOSITIF DESTINÉ À ÊTRE UTILISÉ DANS LA CHIRURGIE DU GLAUCOME

(30) Priorität: 12.01.2012 DE 102012200411
(43) Veröffentlichungstag der Anmeldung: 19.11.2014
(73) Patentinhaber: Geuder AG, 69126 Heidelberg (DE)
(72) Erfinder: HATTENBACH, Lars-Olof, 67061 Ludwigshafen (DE)
(74) Vertreter: Patent- und Rechtsanwälte Ullrich & Naumann
(86) Internationale Anmeldenummer: PCT/DE2013/200001
(87) Internationale Veröffentlichungsnummer: WO 2013/104360

(56) Entgegenhaltungen:
- EP-A1- 0 228 185
- US-A- 5 626 559
- US-A- 5 968 058
- US-A1- 2005 119 737

## Beschreibung

Die Erfindung betrifft eine Vorrichtung zur Verwendung in der Glaukomchirurgie, nämlich zur Implantation in das Auge, zum Zweck des Abbaus eines zu hohen intraokularen Drucks.

Die Vorrichtung dient zur Verwendung in der Glaukomchirurgie, nämlich zur operativen Behandlung des grünen Stars. Unter den Begriff "grüner Star" ist eine Vielzahl von Augenerkrankungen unterschiedlicher Ursache zu subsummieren, die allesamt einen Verlust von Nervenfasern zur Folge haben. Als Folge einer solchen Erkrankung entstehen charakteristische Ausfälle des Gesichtsfeldes und im Extremfall eine Erblindung des Auges. Als wichtigster Risikofaktor wird ein zu hoher Augeninnendruck angesehen.

Viele Menschen leiden an einem erhöhten Augeninnendruck. Entsprechend sind viele von einem Glaukom betroffen, alleine in Deutschland etwa eine Million Menschen.

Neben einer medikamentösen Therapie des Glaukoms stehen unterschiedliche chirurgische Verfahren zur Glaukombehandlung zur Verfügung. Dabei wird regelmäßig - in Abhängigkeit vom konkreten Typ der Erkrankung - der Abfluss des Kammerwassers begünstigt, wodurch eine Senkung des Augeninnendrucks möglich ist. Die Trabekulektomie (TE) ist neben anderen filtrierenden Eingriffen als Standardverfahren der penetrierenden Glaukomchirurgie anzusehen. In der Lederhaut (Sklera) wird eine künstliche Öffnung angelegt, die anschließend locker verschlossen und mit Bindehaut überdeckt wird. Unterschiedliche Shunt-Systeme dienen zur Drainage des Kammerwassers.

Basierend auf dem Prinzip der Trabekulektomie ist die Subkonjunktival-Drainage bekannt, wonach ein Kanalsystem zwischen der Vorderkammer des Auges und einer skleralen Tasche bzw. einem subkonjunktivalen Sickerkissen gebildet wird. Zur Realisierung einer entsprechenden Drainage ist aus der Praxis das Ahmed-Valve bekannt.

Ein weiterer operativer Ansatz ist der Trabekelwerk-Bypass, wonach der Shunt durch die Implantation eines Mikroröhrchens einen offenen Kanal im Trabekelwerk erhält. Aus der Praxis ist hierzu der iStant bekannt.

Eine weitere Methode nutzt eine Drainage in den suprachoroidalen Spalt. Dazu ist aus der Praxis der SOLX Shunt bekannt.

Alle aus der Praxis bekannten Glaukom-Shunts basieren auf der Ableitung von Flüssigkeit aus der Vorderkammer des Auges, siehe beispielsweise die Offenlegungsschrift US5626559. Eine weitere Offenlegungsschrift US 2005/119737 A1 offenbart einen Glaukom-Shunt, welcher zwischen Hinterkammer und einem Spalt zwischen Bindehaut und Sklera, angebracht werden kann. Das Implantat weist ein flaches, konvexes Kopfteil und einen Entlastungskanal zur Drainage des Auges auf.

Die aus der Praxis bekannten Vorrichtungen zum Abbau eines zu hohen intraokularen Drucks sind schwierig in der Handhabung, insbesondere bei der Implantation. Außerdem besteht die Gefahr der Fehlplatzierung und/oder des "Wanderns".

Der vorliegenden Erfindung liegt daher die Aufgabe zugrunde, eine Vorrichtung zur Verwendung in der Glaukomchirurgie, zur Implantation in das Auge, anzugeben, die sich zum Zwecke des Abbaus eines zu hohen intraokularen Drucks möglichst einfach und dabei sicher handhaben und implantieren lässt. Außerdem soll ein sicherer Sitz im implantierten Zustand, d.h. im Auge, gewährleistet sein. Schließlich soll die Vorrichtung preiswert in der Herstellung sein.

Die voranstehende Aufgabe ist durch die Merkmale des Patentanspruchs 1 gelöst. Danach umfasst die erfindungsgemäße Vorrichtung zur Verwendung in der Glaukomchirurgie, nämlich zur Implantation in das Auge, einen flachen Kopfbereich, eine sich an den Kopfbereich anschließende Sonde und einen sich durch den Kopfbereich und die Sonde hindurch erstreckenden, beidseits offenen Entlastungskanal.

Bei dem Kopfbereich handelt es sich um einen äußerst flachen Bereich, der wie ein flacher Nagelkopf oder Kopf einer Reißzwecke ausgebildet ist. So bildet der Kopfbereich mit seiner Unterseite eine Anlagefläche und ermöglicht somit eine exakte Positionierung für die Sonde.

Der Entlastungskanal ist durch ein beidseits offenes Röhrchen gebildet, wobei dieses im Kopfbereich endet, derart, dass sich der Entlastungskanal durch den Kopfbereich hindurch erstreckt und nach außen öffnet. Somit ist der Entlastungskanal an beiden Enden offen.

Der Kopfbereich und die Sonde sind derart dimensioniert und ausgebildet, dass die Sonde zum Zweck des Abbaus eines zu hohen intraokularen Drucks - bei geöffneter Bindehaut - mit dem freien Ende durch die Lederhaut bzw. Sklera hindurch bis in die Hinterkammer des Auges steckbar ist. Dadurch ist die Dimension bzw. Länge der Sonde vorgegeben, wobei in der eingesteckten Position der Kopfbereich mit seiner Unterseite an der Sklera zur Anlage kommt.

Der erfindungsgemäßen Vorrichtung liegt somit die Idee zugrunde, nicht etwa wie im Stand der Technik üblich, Flüssigkeit aus der Vorderkammer des Auges abzuleiten. Ganz im Gegenteil geht es erfindungsgemäß darum, den Glaukom-Shunt dort zu setzen, wo der zu hohe intraokulare Druck wirkt, nämlich in der Hinterkammer des Auges. Entsprechend ist die Vorrichtung mit der Sonde derart dimensioniert, dass sie sich durch die Sklera hindurch bis in die Hinterkammer des Auges stecken lässt, so dass eine Strömungsverbindung zwischen der Hinterkammer des Auges nach außen hergestellt ist. Nach dem Einbringen der Vorrichtung ist die besondere Funktion des Kopfbereichs von Bedeutung, wobei über dem Kopfbereich die Bindehaut wieder geschlossen und regelmäßig vernäht wird. Folglich überdeckt der ursprünglich geöffnete Bindehaut-Flap die Oberseite des Kopfbereichs, so dass eine Drainage von der Hinterkammer in einen Spalt zwischen Bindehaut und Sklera realisiert ist.

An dieser Stelle sein angemerkt, dass es sich bei der erfindungsgemäßen Vorrichtung um eine Art Trokarröhrchen handelt, welches im Sinne eines Implantats zur Drainage aus der Hinterkammer in den Bereich zwischen Bindehaut und Sklera dient.

Um keine Netzhautablösung zu provozieren, bietet sich der Pars-Plana-Bereich zur Implantation an. Nach Präparation bzw. Öffnung der Bindehaut ist es möglich, mit geringstmöglichem Aufwand ein oder auch mehrere Shunts in Form der erfindungsgemäßen Vorrichtung, d.h. in Form von sehr kleinen und flachen Trokaren, unter die Bindehaut zu setzen. Nach dem Setzen der Trokare kann der zur Öffnung erzeugte Bindehaupt-Flap wieder über den Trokarkopf bzw. die Trokarköpfe geschlossen und vernäht werden, so dass unter Nutzung der erfindungsgemäßen Vorrichtung in den Spalt zwischen Bindehaut und Sklera drainiert wird.

In vorteilhafter Weise ist der Kopfbereich zumindest auf der Unterseite, d.h. auf der der Sonde zugewandten Seite, der Geometrie des Auges entsprechend konkav gewölbt. Somit ist ein sicherer Sitz der Vorrichtung unmittelbar an bzw. auf der Sklera möglich. Auch ist es von Vorteil, wenn der Kopfbereich auf der Oberseite, d.h. auf der der Sonde abgewandten Seite, zumindest geringfügig konvex ausgeführt ist. Dadurch lässt sich das Überziehen des Kopfbereichs mit Bindehaut bzw. mit dem Bindehaut-Flap begünstigen.

Der Kopfbereich weist auf der Oberseite die Drainage begünstigende, radiale Riefen oder Rillen auf, um nämlich aus dem Entlastungskanal der Sonde austretende Flüssigkeit, möglichst unter Nutzung von Kapillarkräften, in den Spalt zwischen Sklera und Bindehaut zu führen. Die Drainage ist dadurch abermals begünstigt.

Die Vorrichtung besteht in vorteilhafter Weise aus einem biokompatiblen Material, zumindest ist die Vorrichtung mit biokompatiblem Material beschichtet. Im Rahmen einer konkreten Ausgestaltung kann die Vorrichtung aus Keramik, Glaskeramik oder Glas bestehen. Das Material kann permeabel oder semipermeabel ausgeführt sein, wodurch die Drainagewirkung abermals begünstigt ist.

Wie bereits zuvor ausgeführt, dient die erfindungsgemäße Vorrichtung zur Verwendung in der Glaukomchirurgie, dort im Konkreten zur Implantation in das Auge zum Zweck des Abbaus eines zu hohen intraokularen Drucks. Entsprechend ist auch ein Verfahren zur Verwendung einer erfindungsgemäßen Vorrichtung beschrieben, nämlich in der Glaukomchirurgie zur Implantation in das Auge, und zwar zum Zweck des Abbaus eines zu hohen intraokularen Drucks. Dabei ist wesentlich, dass die Sonde bei geöffneter Bindehaut mit dem freien Ende durch die Sklera hindurch bis in die Hinterkammer des Auges gesteckt wird, wobei bei an der Sklera anliegendem Kopfbereich dieser durch Schließen der Bindehaupt bzw. des beim Öffnen der Bindehaut erzeugten Bindehaut-Flaps überdeckt wird, so dass eine Drainage aus der Hinterkammer heraus in den Spalt zwischen Bindehaut und Sklera realisiert ist.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In den Figuren zeigen
- Fig. 1: in einer schematischen Teilansicht ein menschliches Auge im Pars-Plana-Bereich bei geöffneter Bindehaut und bereits eingesteckter erfindungsgemäßer Vorrichtung, und
- Fig. 2: in einer schematischen Teilansicht das Auge gemäß Fig. 1, wobei die Bindehaut durch den beim Öffnen erzeugten Bindehaut-Flap über den Trokarkopf hinweg bereits wieder geschlossen ist.

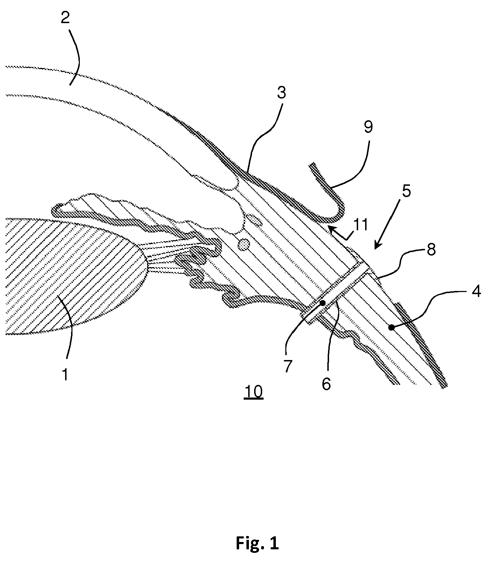

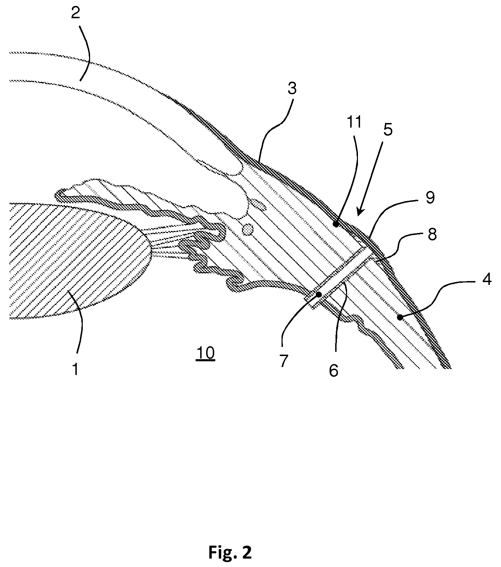

Die Figuren 1 und 2 zeigen in einer schematischen Teilansicht, geschnitten, in vereinfachter Darstellung das menschliche Auge, wobei dort die Linse 1, die Cornea 2, die Bindehaut 3 und die Sklera 4 als wesentliche Bestandteile bezeichnet sind.

Bei der Darstellung in Fig. 1 ist die erfindungsgemäße Vorrichtung 5 bei geöffneter Bindehaut 3 im Sinne eines Implantats eingesetzt, wobei die Sonde 6 komplett durch die Sklera 4 hindurch ragt. Durch die Sonde 6 hindurch erstreckt sich der Entlastungskanal 7, der beidseitig offen ist.

Der Einschub der Vorrichtung 5 bzw. der Sonde 6 ist durch den Kopfbereich 8 begrenzt. Genauer gesagt liegt der Kopfbereich 8 mit seiner Unterseite an der Sklera 4 dicht an.

Gemäß der Darstellung in Fig. 2 ist der zum Öffnen der Bindehaut 3 angelegte Flap 9 über den Kopfbereich 8 der Vorrichtung 5 gelegt und ist die Bindehaut 3 vernäht. Die Vorrichtung 5 ist somit insgesamt implantiert, wodurch eine Drainage zwischen der Hinterkammer 10 und einem Spalt 11 zwischen Sklera 4 und Bindehaut 3 geschaffen ist. Letztendlich ist unter Verwendung der erfindungsgemäßen Vorrichtung eine Druckentlastung aus der Hinterkammer 10 heraus geschaffen.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die Ansprüche verwiesen.

Schließlich sei ausdrücklich darauf hingewiesen, dass das voranstehend beschriebene Ausführungsbeispiel der erfindungsgemäßen Vorrichtung lediglich zur Erörterung der beanspruchten Lehre dient, diese jedoch nicht auf das Ausführungsbeispiel einschränkt.

### Bezugszeichenliste

- 1: Linse
- 2: Cornea
- 3: Bindehaut
- 4: Sklera,Lederhaut
- 5: Vorrichtung (umfassend Sonde 6, Entlastungskanal 7 und Kopfbereich 8)
- 6: Sonde
- 7: Entlastungskanal
- 8: Kopfbereich
- 9: Flap (der Bindehaut)
- 10: Hinterkammer (des Auges)
- 11: Spalt (zwischen Sklera und Bindehaut)

## Patentansprüche

1. Vorrichtung zur Verwendung in der Glaukomchirurgie, nämlich zur Implantation in das Auge, mit einem Kopfbereich (8) und einer sich an den Kopfbereich (8) anschließenden Sonde (6),
**dadurch gekennzeichnet, dass** sich durch den Kopfbereich (8) und die Sonde (6) hindurch ein beidseits offener Entlastungskanal (7) erstreckt, wobei der Kopfbereich (8) und die Sonde (6) derart dimensioniert und ausgebildet sind, dass die Sonde (6) zum Zweck des Abbaus eines zu hohen intraokularen Drucks bei geöffneter Bindehaut (3) mit dem freien Ende durch die Sklera (4) hindurch bis in die Hinterkammer (10) des Auges steckbar ist, dass in der eingesteckten Position der Kopfbereich (8) mit seiner Unterseite an der Sklera (4) zur Anlage kommt und durch Schließen der Bindehaut (3) die Oberseite des Kopfbereichs (8) überdeckbar ist, so dass eine Drainage von der Hinterkammer (10) in einen Spalt (11) zwischen Bindehaut (3) und Sklera (4) realisierbar ist und dass der Kopfbereich (8) flach ausgebildet ist und auf der Oberseite die Drainage begünstigende Riefen oder Rillen aufweist.

2. Vorrichtung nach Anspruch 1, **dadurch gekennzeichnet, dass** der Kopfbereich (8) zumindest auf der Unterseite, d.h. auf der der Sonde (6) zugewandten Seite, der Geometrie des Auges entsprechend konkav gewölbt ausgeführt ist.

3. Vorrichtung nach Anspruch 1 oder 2, **dadurch gekennzeichnet, dass** der Kopfbereich (8) auf der Oberseite, d.h. auf der der Sonde (6) abgewandten Seite, zumindest geringfügig konvex ausgeführt ist.

4. Vorrichtung nach einem der Ansprüche 1 bis 3, **gekennzeichnet durch** eine Beschichtung und/oder Herstellung aus einem biokompatiblen Material.

5. Vorrichtung nach einem der Ansprüche 1 bis 4, **gekennzeichnet durch** die Herstellung aus Keramik, Glaskeramik oder Glas.

6. Vorrichtung nach Anspruch 4 oder 5, **dadurch gekennzeichnet, dass** das Material permeabel oder semipermeabel ausgeführt ist.

## Claims

1. Device for use in glaucoma surgery, in particular for implantation in the eye, comprising a head region (8) and a probe (6) that adjoins the head region (8), **characterised in that** a relief channel (7), which is open on both sides, extends through the head region (8) and the probe (6), the head region (8) and the probe (6) being dimensioned and configured in such a way that, when the conjunctiva (3) is open, the free end of the probe (6) can be inserted through the sclera (4) as far as the posterior chamber (10) of the eye for the purpose of lowering an abnormally high intraocular pressure, **in that**, in the inserted position, the bottom side of the head region (8) comes to rest against the sclera (4), and the upper side of the head region (8) can be covered by closing the conjunctiva (3) so that drainage from the posterior chamber (10) into a gap (11) between the conjunctiva (3) and the sclera (4) can be achieved, and **in that** the head region (8) is flat and has grooves or notches on the upper side facilitating drainage.

2. Device according to claim 1, **characterised in that** the head region (8) is concavely curved at least on the bottom side, i.e. on the side facing the probe (6), in conformity with the geometry of the eye.

3. Device according to either claim 1 or claim 2, **characterised in that** the head region (8) is at least slightly convex on the upper side, i.e. on the side facing away from the probe (6).

4. Device according to any one of claims 1 to 3, **characterised by** said device being coated with and/or made of a biocompatible material.

5. Device according to any one of claims 1 to 4, **characterised by** said device being made of ceramic, glass ceramic, or glass.

6. Device according to either claim 4 or claim 5, **characterised in that** the material is permeable or semi-permeable.

## Revendications

1. Dispositif destiné à être utilisé dans la chirurgie du glaucome, à savoir pour une implantation intraoculaire, avec une zone de tête (8) et une sonde (6) se raccordant à la zone de tête (8),
**caractérisé en ce qu'**un conduit de détente (7) ouvert des deux côtés s'étend à travers la zone de tête (8) et la sonde (6), la zone de tête (8) et la sonde (6) étant dimensionnées et constituées de telle sorte que, dans le but de réduire une pression intraoculaire trop élevée, la sonde (6) peut, alors que la conjonctive (3) est ouverte, être introduite par l'extrémité libre à travers la sclérotique (4) jusque dans la chambre postérieure (10) de l'oeil, **en ce que**, dans la position introduite, la zone de tête (8) vient par son côté inférieur en appui sur la sclérotique (4), et le côté supérieur de la zone de tête (8) peut être recouvert par la fermeture de la conjonctive (3) de telle sorte qu'un drainage de la chambre postérieure (10) peut être réalisé dans un interstice (11) entre la conjonctive (3) et la sclérotique (4), et **en ce que** la zone de tête (8) est constituée de façon plate et présente sur le côté supérieur des cannelures ou rainures favorisant le drainage.

2. Dispositif selon la revendication 1, **caractérisé en ce que** la zone de tête (8), au moins sur le côté inférieur, c'est-à-dire sur le côté tourné vers la sonde (6), est réalisée avec un bombement concave conformément à la géométrie de l'oeil.

3. Dispositif selon la revendication 1 ou 2, **caractérisé en ce que** la zone de tête (8), sur le côté supérieur, c'est-à-dire sur le côté éloigné de la sonde (6), est réalisée de façon au moins légèrement convexe.

4. Dispositif selon l'une des revendications 1 à 3, **caractérisé par** un revêtement et/ou une fabrication en matériau biocompatible.

5. Dispositif selon l'une des revendications 1 à 4, **caractérisé par** la fabrication en céramique, vitrocéramique ou verre.

6. Dispositif selon la revendication 4 ou 5, **caractérisé en ce que** le matériau est réalisé de façon perméable ou semi-perméable.
